(19) Europäisches Patentamt
European Patent Office
Office européen des brevets

(11) **EP 1 991 116 B1**

(12) **EUROPÄISCHE PATENTSCHRIFT**

(45) Veröffentlichungstag und Bekanntmachung des Hinweises auf die Patenterteilung:
**19.06.2019 Patentblatt 2019/25**

(21) Anmeldenummer: **07721981.4**

(22) Anmeldetag: **05.03.2007**

(51) Int Cl.:
*A61B 5/021* (2006.01)    *A61B 5/0285* (2006.01)
*A61M 1/14* (2006.01)

(86) Internationale Anmeldenummer:
**PCT/DE2007/000406**

(87) Internationale Veröffentlichungsnummer:
**WO 2007/101431 (13.09.2007 Gazette 2007/37)**

(54) **DIALYSEGERÄT MIT MESSEINRICHTUNGEN ZUR ERFASSUNG DES BLUTDRUCKS SOWIE VERFAHREN ZUR BESTIMMUNG DES BLUTDRUCKS SOWIE EIN SPEICHERMEDIUM ZUR VERWENDUNG IN EINEM DIALYSEGERÄT**

DIALYSIS APPLIANCE WITH MEASURING MEANS FOR RECORDING BLOOD PRESSURE, AND METHOD FOR DETERMINING THE BLOOD PRESSURE, AND A STORAGE MEDIUM FOR USE IN A DIALYSIS APPLIANCE

DIALYSEUR MUNI DE DISPOSITIFS DE MESURE POUR L'ACQUISITION DE LA PRESSION SANGUINE ET PROCÉDÉ DE DÉTERMINATION DE LA PRESSION SANGUINE ET SUPPORT D'ENREGISTREMENT À UTILISER DANS UN DIALYSEUR

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **07.03.2006 DE 102006010813**

(43) Veröffentlichungstag der Anmeldung:
**19.11.2008 Patentblatt 2008/47**

(73) Patentinhaber: **Fresenius Medical Care Deutschland GmbH**
**61352 Bad Homburg (DE)**

(72) Erfinder: **ZHANG, Wei**
**97464 Niederwerrn (DE)**

(74) Vertreter: **Herrmann, Uwe et al**
**Lorenz Seidler Gossel**
**Rechtsanwälte Patentanwälte**
**Partnerschaft mbB**
**Widenmayerstraße 23**
**80538 München (DE)**

(56) Entgegenhaltungen:
**EP-A- 0 875 200    EP-A1- 0 911 044**
**EP-A2- 1 199 029    DE-A1- 10 114 383**
**US-A- 5 876 348    US-A1- 2002 193 691**

**Beschreibung**

Die Erfindung betrifft eine Vorrichtung bestehend aus einem Dialysegerät mit einer dem Dialysegerät zugeordneten Messeinheit zur Erfassung des Blutdrucks, mit einer dem Dialysegerät zugeordneten Messeinrichtung zur Erfassung einer Pulswellenlaufzeit sowie mit einer Auswerteeinheit, nach dem Patentanspruch 1 sowie ein Verfahren zur Bestimmung der Parameter der Beziehung zwischen einem Pulswellenlaufzeitsignal und einem Blutdruckmesssignal nach Patentanspruch 7.

Es sind bereits derartige Dialysegeräte bekannt, bei denen während der Dialysebehandlung von dem Gerät selbsttätig der Blutdruck gemessen wird. Als Dialysegeräte werden vorliegend sämtliche Geräte zur Nierenersatzbehandlung verstanden, die in den Flüssigkeitshaushalt eines Patienten eingreifen, unabhängig davon, ob es sich z. B. um eine Hämodialyse-, Hämofiltrations- oder Hämodiafiltrationsgerät handelt.

Blutdruckabfälle während der Dialysebehandlung gehören zu den häufigsten Komplikationen. Sie kommen bei etwa 20 - 30% der Behandlung vor. Eine solche hypotone Episode kommt auch oft plötzlich vor und kann einerseits bis zur Bewusstlosigkeit des Patienten und anderseits zum Abbruch der Behandlung und sogar zur teueren, aufwendigen Interventionen führen. Dadurch werden die Morbidität und Mortalität eines Patienten sehr negativ beeinflusst.

Zur Aufrechterhaltung eines konstanten Blutdrucks sind mehrere Regulationsmechanismen notwendig. Diese können sowohl durch Hämodialysebehandlung als auch durch die diversen Vor- und Begleiterkrankungen des Patienten beeinträchtigt werden.

Durch Ultrafiltration kommt es zu einer Abnahme des Plasmavolumens. Falls der Organismus es nicht schafft, das Plasmavolumen aus dem interstitiellen Raum aufzufüllen, sinkt der Füllungsdruck des Herzens, und der Blutdruck fällt. Dies wird umso eher geschehen, wenn hohe Ultrafiltrationsraten erforderlich sind. Gehäuft kommen die Blutdruckabfälle bei Patienten mit eingeschränkten Herzkreislauf-Funktionen vor, wie bei älteren Menschen, bei Patienten mit Myokardinsuffizienz, diabetischer Nephropathie und bei Einnahme entsprechend Kreislauf-wirksamer Medikamente.

Um den Abfall des Blutdrucks frühzeitig zu erkennen und/oder zu vermeiden, sind verschiedene Wirkungsweisen von Dialysegeräten bekannt. Zum einen gibt es Monitore, die das relative Blutvolumen während der Dialysebehandlung messen und ggf. die Ultrafiltration regeln. Zum anderen gibt es Monitore, die Blutdruckänderungen eines Patienten durch die Erfassung der Pulswellenlaufzeit (PTT) kontinuierlich überwachen und ggf. die Ultrafiltration regeln. Die Zeit eines Druckpulses, die dieser zwischen zwei Punkten entlang eines Gefäßes im Patienten benötigt, ist eine Funktion des Blutdrucks. Diese Zeit lässt sich vergleichsweise einfach ermitteln. Als Startsignal kann ein EKG-Puls verwendet werden., während als Stopp-Signal ein optisches Pulsmessgerät an einer vom Herz entfernten Peripheriestelle verwendet werden kann. Diese Vorgehensweise ist bereits in der US-PS 6,736,789 beschrieben, auf deren Offenbarungsgehalt explizit Bezug genommen wird.

Eine solche Vorgehensweise ist ebenfalls in der US 2002/0193691 A1 beschrieben. Dort wird die Pulswellengeschwindigkeit ausgewertet, die aber unmittelbar mit der Pulswellenlaufzeit zusammenhängt. Es besteht außerdem ein linearer Zusammenhang zwischen dem Blutdruck und der Pulswellengeschwindigkeit (Pulswellenlaufzeit). In einer mathematischen Formel lässt sich dieser Zusammenhang in Form einer Geradengleichung ausdrücken. Liegen zwei Messwertepaare vor von zeitgleich erfasstem Blutdruck und Pulswellengeschwindigkeit (Pulswellenlaufzeit), lassen sich die beiden Parameter der Geradengleichung bestimmen. Wenn die Parameter bestimmt sind, kann im weiteren aus der Messung der Pulswellengeschwindigkeit (Pulswellenlaufzeit) mit der Geradengleichung und den dann bekannten Parametern der Blutdruck ermittelt werden. Bei der US 2002/0193691 A1 wird der Wert des Blutdrucks, der mit der Geradengleichung aus der Pulswellengeschwindigkeit ermittelt wird, darauf überwacht, ob der Blutdruck übermäßig stark abfällt. In diesem Fall werden bestimmte medizinisch notwendige Maßnahmen eingeleitet. In der US 2002/0193691 A1 ist außerdem beschrieben, dass aus dem Verfahrensablauf, in dem das Unterschreiten eines Schwellwertes des Blutdrucks überwacht wird, in einen weiteren Verfahrensablauf abgezweigt wird, in dem die Parameter der Geradengleichung bestimmt werden.

Aus der US-PS 5,876,248 ist es bekannt, den Blutdruck mittels der beschriebenen Geradengleichung aus der Pulswellenlaufzeit zu ermitteln. Bei der US-PS 5,876,248 wird bei Unterschreiten des Wertes des Blutdruckes, der über die Geradengleichung aus der Pulswellenlaufzeit abgeleitet wurde, eine Messung des Blutdrucks eingeleitet. Eine derartige Messung kann beispielsweise mit einer Manschette erfolgen. Bei diesem Patent geht es also darum, einen Abfall des Blutdrucks festzustellen. Wenn dieser Abfall festgestellt wurde, wird eine Messung des Absolutwertes des Blutdrucks mit einer Manschettenmessung vorgenommen. Die Bestimmung der Parameter zur Berechnung des Blutdrucks aus Messwerten der Pulswellenlaufzeit erfolgt, indem zur Geradengleichung ein erstes Messwertepaar aufgenommen wird in einem Ruhezustand des Patienten und ein zweites Messwertepaar bei einer Bewegung des Patienten.

Aus der DE 101 14 383 A1 ist es ebenfalls bekannt, die Parameter der Geradengleichung zu dem Zusammenhang zwischen dem Blutdruck und der Pulswellenlaufzeit zu bestimmen. Um ein Maß für eine kritische Änderung des Blutdrucks ableiten zu können, wird dort nicht nur der Blutdruck und dessen Änderung dem Betrag nach ausgewertet sondern auch die Änderungsgeschwindigkeit des Blutdrucks.

Aus der US-PS 6,852,083 ist ein Verfahren zur wiederholten Kalibrierung von Parametern bekannt, bei dem aus dem abgeleiteten Signal (bei dem der Blutdruck beispielsweise aus der Pulswellenlaufzeit abgeleitet wird), ein Triggersignal bestimmt wird. Überschreitet dieses Triggersignal einen bestimmten Schwellwert, wird eine erneute Kalibrierung vorgenommen. Wie die Bedingung dieses Triggersignals erreicht wird, ist in der US-PS 6,852,083 nicht erläutert.

Aus der US-PS 5,603,329 ist es bekannt, aus gemessenen Werten des Blutdrucks zu bestimmten Zeitpunkten und der Pulswellenlaufzeit die Parameter einer linearen Gleichung zu bestimmen. Dazu wird ein Zeitpunkt einer systolischen Pulswelle ermittelt zusammen mit dem zugehörigen Wert des Blutdrucks. Weiterhin wird der Zeitpunkt der diastolischen Pulswelle ermittelt, die zu der systolischen Pulswelle gehört. Zu diesem Zeitpunkt wird ebenfalls wieder der zugehörige Wert des Blutdrucks ermittelt. Aus diesen beiden Messwertepaaren werden die Parameter der linearen Gleichung bestimmt, die den Zusammenhang zwischen der Pulswellenlaufzeit und dem Blutdruck beschreiben. Nachdem diese Parameter bestimmt sind, kann aus der gemessenen Pulswellenlaufzeit unter Verwendung dieser Parameter der Blutdruck bestimmt werden. Die Steigung der Geraden kann weiterhin verwendet werden, um eine Aussage über den Grad der Arteriosklerose bei dem Patienten zu treffen.

Aus der US-PS 5,876,348 ist es bekannt, den Blutdruck mit der angesprochenen Geradengleichung zu bestimmen und die Änderung des Blutdrucks auf einen bestimmten Schwellwert zu überwachen. Die Parameter der Gleichung können von einem externen Speichermedium eingelesen werden. Ebenso ist beschrieben, dass Wertepaare des Blutdrucks und der Pulswellenlaufzeit aufgenommen werden. Zur Bestimmung der Parameter der Geradengleichung müssen wenigstens zwei Wertepaare aufgenommen werden. Im Weiteren können aus diesen Wertepaaren mit der Methode der kleinsten Fehlerquadrate die Parameter der Geradengleichung bestimmt werden.

Die beiden Wertepaare zur Bestimmung der Parameter der Geradengleichungen sollen zum einen aufgenommen werden, wenn der Patient ruht, und zum anderen, wenn der Patient in Bewegung ist. Damit soll erreicht werden, dass die jeweils zueinander gehörenden Werte der beiden Wertepaare, deren Differenzen gebildet werden müssen zur Bestimmung der Parameter, so weit auseinander liegen, dass statistische Fehler bei der Messung vernachlässigt werden können. Es ist auch ausgeführt, dass es nicht zwingend auf den Ruhe- bzw. Bewegungszustand des Patienten ankommt, wenn die entsprechenden Bedingungen anderweitig erreicht werden.

In der EP 0 875 200 A1 ist insoweit dasselbe beschrieben wie in der US-PS 5, 876,348. Außerdem ist dort beschrieben, dass der Blutdruck aus der Pulswellenlaufzeit errechnet wird unter Verwendung eines ersten Datensatzes der Parameter der Geradengleichung. Wenn die Änderung der Pulswellenlaufzeit zwischen zwei Messungen den kritischen Schwellwert für die Erkennung eines Blutdruckabfalls überschreitet, erfolgt eine Blutdruckmessung über eine Manschette. Dieses Wertepaar der gemessenen Pulswellenlaufzeit und des mit der Manschette gemessenen Blutdrucks wird bei dem erkannten Blutdruckabfall verwendet wird, um die Parameter der Geradengleichung neu zu berechnen.

Gleichung (1) stellt ein Ausführungsbeispiel für eine lineare Darstellung des Zusammenhangs zwischen dem BP und der PTT dar,

$$BP(t) = m - n \cdot PTT(t) \qquad (1)$$

wobei m und $n$ patientenabhängige Konstanten sind, welche sich über einen langen Zeitraum verändern können. Aus Gleichung (1) folgt

$$\frac{BP(0) - BP(i)}{BP(0)} = K \cdot \frac{PTT(i) - PTT(0)}{PTT(0)} \qquad (2)$$

$$K = \frac{n \cdot PTT(0)}{m - n \cdot PTT(0)} \qquad (3)$$

wobei

BP(0) - gemessener Blutdruck (mmHg) bei Dialysezeit $t_0$
PTT(0) - gemessene Pulswellenlaufzeit (ms) bei Dialysezeit $t_0$
BP(i) - gemessener Blutdruck (mmHg) bei Dialysezeit $t_i$
PTT(i) - gemessene Pulswellenlaufzeit (ms) bei Dialysezeit $t_i$

Gleichung (2) bedeutet, dass die relative PTT-Änderung über Parameter K mit der relativen BP-Änderung verbunden ist und K von m und $n$ abhängig ist. Die Konstanten m und $n$ müssen durch zwei Kalibrierungsmessungen ermittelt werden, um eine präzise Umrechnung von der relativen PTT-Änderung zur BP-Änderung zu ermöglichen.

Weiterhin ist es bekannt, den Blutdruck mittels einer konventionellen Manschettenmessung zu überwachen. Hierbei kann es für den Patienten als unangenehm empfunden werden, dass in regelmäßigen Abständen (z. B. alle 30 Minuten) die Manschette mit Druck beaufschlagt werden muss, um die Messung durchführen zu können. Ein weiterer und entscheidender Nachteil der Manschettenmessung liegt darin, dass ein rasanter Blutdruckabfall wegen der Diskontinuität der Messung schwer zu erfassen ist.

Der vorliegenden Erfindung liegt das Problem zu Grunde, eine Vorgehensweise für eine einfache und für den Patienten komfortable Messung des absoluten Blutdrucks bzw. der Blutdruckänderung vorzuschlagen. Dabei soll der Blutdruck aus der Pulswellenlaufzeit abgeleitet werden. Der vorliegenden Erfindung liegt die Aufgabe zu Grunde, die Genauigkeit der Bestimmung des Blutdrucks aus der Pulswellenlaufzeit zu verbessern.

Diese Aufgabe wird nach der vorliegenden Erfindung durch eine Vorrichtung gemäß Anspruch 1 gelöst, die aus einem Dialysegerät besteht sowie einer dem Dialysegerät zugeordneten Messeinheit zur Erfassung des Blutdrucks, einer dem Dialysegerät zugeordneten Messeinrichtung zur Erfassung einer Pulswellenlaufzeit sowie einer Auswerteeinheit. Dabei ist die Auswerteeinheit so konfiguriert, dass die Parameter der Abhängigkeit der Pulswellenlaufzeit zum Blutdruck aus Messwerten der Messeinheit zur Erfassung des Blutdrucks zu zeitgleichen Messwerten der Messeinrichtung zur Erfassung der Pulswellenlaufzeit bestimmt werden. Nach der vorliegenden Erfindung ist die Vorrichtung so konfiguriert, dass wenigstens zwei dieser Messwertepaare zu Zeitpunkten ermittelt werden, bei denen die absolute und/oder die relative Abweichung der Pulswellenlaufzeit oberhalb eines Schwellwertes liegt, indem zu einem ersten Zeitpunkt ein erstes Messwertepaar ermittelt wird, wobei die Vorrichtung so konfiguriert ist, dass der zweite Zeitpunkt ermittelt wird, indem die Messwerte der Messeinrichtung zur Erfassung der Pulswellenlaufzeit gegenüber dem Messwert der Messeinrichtung zur Erfassung der Pulswellenlaufzeit zu dem ersten Zeitpunkt daraufhin überwacht werden, ob die absolute und/oder relative Abweichung oberhalb des Schwellwertes liegt. Die Vorrichtung ist so konfiguriert, dass bei der Feststellung, dass die absolute und/oder relative Abweichung oberhalb des Schwellwertes liegt, die Ermittlung des zweiten Messwertepaares erfolgt. Die Vorrichtung ist dazu konfiguriert, ein erstes der zwei Messwertpaare vor einer Blutentnahme zu Beginn einer Dialysebehandlung zu ermitteln und ein zweites der zwei Messwertpaare nach Füllung eines extrakorporalen Kreislaufs mit entnommenem Blut zu ermitteln.

Unter der absoluten und/oder relativen Abweichung wird dabei im Rahmen dieses Schutzrechtes in weiterer vorteilhafter Ausgestaltung der stets positive Betrag der Abweichung verstanden. Damit kann auch das Unterschreiten eines unteren Grenzwertes bei nicht betragsmäßiger Betrachtung im gleichgelagerten Fall des starken Absinkens der Messwerte in einfacher Weise mit einbezogen werden.

Dabei erweist es sich als vorteilhaft, dass zur Bestimmung der Parameter der Beziehung zumindest zwischen zwei Messwertepaaren eine hinreichend große Differenz auch der Werte des Blutdrucks vorliegt, so dass davon ausgegangen werden kann, dass insbesondere auch die Parameter mit ausreichender Genauigkeit bestimmbar sind.

Die Abhängigkeit der Pulswellenlaufzeit zum Blutdruck kann beispielsweise als lineare Abhängigkeit in Form einer Geraden parametriert werden.

Sofern auf Grund von vorher gehenden Behandlungen bereits Parameter bestimmt sind, kann aus den gemessenen Pulswellenlaufzeiten mit diesen Parametern ein Wert des Blutdrucks ermittelt werden. Die Bedingung zur Erfassung weiterer Messwertepaare kann in diesem Falle hinsichtlich des Schwellwertes auf den Blutdruck selbst als aus der gemessenen Pulswellenlaufzeit abgeleitete Größe bezogen werden.

Sofern noch keine Parameter bestimmt worden sind, kann ebenfalls ein Schwellwert vorgesehen werden, der sich auf die Pulswellenlaufzeit bestimmt. Dies kann eine absolute Differenz der Pulswellenlaufzeiten sein oder auch eine relative Abweichung der Pulswellenlaufzeiten.

Bei der vorgeschlagenen Lösung wird die Pulswellenlaufzeit bestimmt und auf den Schwellwert überwacht. Sofern die entsprechende Bedingung vorliegt, wird eine Messung des Blutdrucks mittels der Messeinheit veranlasst. Diese Ausgestaltung hat den Vorteil, dass es nicht notwendig ist, den Blutdruck regelmäßig zu erfassen, um beispielsweise nach Abschluss einer Messphase die Parameter bestimmen zu können. Wenn bei der vorgeschlagenen Ausgestaltung die Bedingung für die Pulswellenlaufzeit vorliegt, wird die entsprechende Messung des Blutdrucks initiiert.

Außer den so abgeleiteten Messwertepaaren können während einer Dialysebehandlung noch weitere Messwertepaare aufgenommen werden. Die Parameter der Beziehung können in diesem Fall aus einem überbestimmten Gleichungssystem ermittelt werden mittels statistischer Methoden wie beispielsweise der Methode der kleinsten Fehlerquadrate.

Bei einem Dialysegerät wird eine erste Messung vorteilhaft zu Beginn der Behandlung vorgenommen. Zu diesem Zeitpunkt ist der Blutdruck hoch, weil das Blut zur Behandlung noch nicht entnommen wurde. Der Eintritt der Bedingung der Schwellwerte der Pulswellenlaufzeit bzw. des Blutdrucks stellt sich dann während der Behandlung erwartungsgemäß von selbst ein, wenn Blut entnommen wird und dadurch der Blutdruck abfällt. Hier erweist es sich als besonders vorteilhaft, die Abhängigkeit der Bestimmung der Parameter von den Schwellwerten vorzusehen.

Es hat sich gezeigt, dass diese durch die Schwellwerte bestimmten Bedingungen mit einer ersten Messung vor der Blutentnahme und wenigstens einer weiteren Messung nach Füllung des extrakorporalen Kreislaufs mit entnommenem Blut erreicht werden.

Da ein Dialysegerät auch Mittel zum Entziehen von Flüssigkeit aus dem extrakorporalen Blut des Patienten aufweist, stellen sich die Schwellwerte erfüllende Bedingungen auch während der Dialyse ein. Gleiches gilt nach Rückgabe des extrakorporalen Blutes nach Beendigung der Dialysebehandlung. Es müssen also durchweg keine weiteren besonderen Maßnahmen getroffen werden.

Die Auswerteeinheit kann dabei so konfiguriert sein, dass Messwerte der Pulswellenlaufzeit sowie des Blutdrucks gezielt vor dem Anschließen eines Patienten an den extrakorporalen Kreislauf, nach dem Anschließen des Patienten und der Befüllung des extrakorporalen Kreislaufs mit Blut vor Beginn der Dialysebehandlung, am Ende der Dialysebehandlung und/oder nach Rückgabe des extrakorporalen Blutes an den Patienten nach Beendigung der Dialysebehandlung aufgenommen werden können. Bei allen diesen Zeitpunkten gibt es dialyseinhärent Veränderungen des Blutdrucks, wodurch sich diese Zeitpunkte besonders zur Erfüllung des Schwellenkriteriums eignen. Dabei ist die Auswerteeinheit zweckmäßigerweise mit einer entsprechend konfigurierten Steuereinheit des Dialysegerätes verbunden, die auch mit den Mitteln zum Entziehen von Flüssigkeit verbunden ist. Es kann dabei auch vorgesehen sein, dass die Mittel zum Entziehen von Flüssigkeit neben dem therapeutischen Entziehen von Flüssigkeit gezielt Flüssigkeit entziehen, um eine entsprechende Blutdruckänderung auf Anforderung herbei zu führen.

Gegebenenfalls kann dazu auch auf einer Ausgabeeinheit wie einem Display oder einer Sprachausgabe die Anweisung ausgegeben werden, dass der Patient umzulagern ist (von der Rückenlage in die Seitenlage oder umgekehrt), um so eine Änderung des Blutdrucks zu bewirken. Diese Maßnahme kann zusätzlich unterstützend vorgesehen werden, wobei sich gezeigt hat, dass die Schwellwerte bereits durch die Dialysebehandlung an sich überschritten werden.

Bei der Ausgestaltung nach Anspruch 2 werden bei der Parameterbestimmung die Messwertepaare entsprechend den Zeitpunkten gewichtet berücksichtigt, zu denen die Messwertepaare aufgenommen wurden, wobei bei länger zurückliegenden Zeitpunkten, zu denen die Messwertepaare aufgenommen wurden, die Gewichtung schwächer ist.

Indem sich die Parameter mit der Zeit ändern können, wird durch die Vorgehensweise nach Anspruch 2 sicher gestellt, dass aktuelle Messwerte eine stärkere Gewichtung erfahren bei der Bestimmung der Parameter. Die Parameter sind dadurch hinreichend aktuell.

Bei der Ausgestaltung nach Anspruch 3 werden zur Bestimmung der Parameter der Beziehung zwischen der Pulswellenlaufzeit und dem Blutdruck der gemessene maximale Wert des Blutdrucks sowie der gemessene minimale Wert des Blutdrucks mit den zugehörigen gemessenen Pulswellenlaufzeiten herangezogen.

Dadurch ergibt sich eine vergleichsweise einfache Bestimmung der Parameter aus den Minimal- und Maximalwerten.

Bei der Ausgestaltung nach Anspruch 4 werden zur Bestimmung der Parameter der Beziehung nur solche Messwertepaare verwendet, die innerhalb eines bestimmten Zeitfensters ermittelt wurden.

Dies ist eine besondere Form der Gewichtung der Messwertepaare abhängig von den Zeitpunkten, zu denen die Messwertepaare ermittelt wurden. Ab einer bestimmten Zeitspanne, zu der die Messwerte zurück liegen, wird deren "Gewichtung" auf Null gesetzt, so dass diese Messwertepaare keine Berücksichtigung mehr finden.

Anspruch 5 betrifft eine Ausgestaltung, bei der dem Dialysegerät eine Dateneinleseeinheit zugeordnet ist bzw. zuordnbar ist, mit der der Auswerteeinheit Messwertepaare der Messeinheit und der Messeinrichtung von früheren Behandlungen desselben Patienten von einem Speichermedium zugeführt werden.

Die Dateneinleseeinheit kann fest an dem Dialysegerät angeschlossen sein. Es ist auch möglich, einen Port für den Anschluss einer portablen Datenleseeinheit vorzusehen, die dann für mehrere Dialysegeräte nacheinander verwendet werden kann.

Bei dieser Lösung müssen die Daten vorteilhaft nicht in der Praxis der Dialysebehandlung auf einem Rechner vorgehalten werden. Diese können der Patientenakte zugeordnet werden, indem dort das Speichermedium aufbewahrt wird. Bei der nächsten Behandlung können die entsprechenden Daten dann einfach wieder zur Verfügung gestellt werden.

Bei der Ausgestaltung nach Anspruch 6 ist die Dateneinleseeinheit zugleich auch eine Datenausleseeinheit, mit der Messwertepaare der aktuellen Behandlung auf das Speichermedium geschrieben werden.

Vorteilhaft stehen diese Daten dann auch wieder bei künftigen Behandlungen zur Verfügung. Bei der Ausgestaltung eines Verfahrens zur Bestimmung der Parameter der Beziehung zwischen einem Pulswellenlaufzeitsignal und einem Blutdruckmesssignal werden gemäß Anspruch 7 die Parameter der Beziehung zwischen der Pulswellenlaufzeit und dem Blutdruck aus Messwerten des Blutdruckmesssignals und zeitgleichen Messwerten der Pulswellenlaufzeit bestimmt. Nach der vorliegenden Erfindung werden wenigstens zwei dieser Messwertepaare zu Zeitpunkten ermittelt, bei denen die absolute und/oder die relative Abweichung der Pulswellenlaufzeit oberhalb eines Schwellwertes liegt, indem zu einem ersten Zeitpunkt ein erstes Messwertepaar ermittelt wird, wobei der zweite Zeitpunkt ermittelt wird, indem die Messwerte der Messeinrichtung zur Erfassung der Pulswellenlaufzeit gegenüber dem Messwert der Messeinrichtung zur Erfassung der Pulswellenlaufzeit zu dem ersten Zeitpunkt daraufhin überwacht werden, ob die absolute und/oder relative Abweichung oberhalb des Schwellwertes liegt, wobei bei der Feststellung, dass die absolute und/oder relative Abweichung oberhalb des Schwellwertes liegt, die Ermittlung des zweiten Messwertepaares erfolgt, wobei die Ermittlung eines eines ersten der zwei Messwertpaare vor einer Blutentnahme zu Beginn einer Dialysebehandlung erfolgt und die Ermittlung eines zweiten der zwei Messwertpaare nach Füllung eines extrakorporalen Kreislaufs mit entnommenem Blut erfolgt.

Dabei erweist es sich als vorteilhaft, dass zur Bestimmung der Parameter der Beziehung zumindest zwischen zwei Messwertepaaren auch eine hinreichend große Differenz der Werte des Blutdrucks vorliegt. Sofern mit den Parametern eine Gerade definiert wird, kann davon ausgegangen werden, dass insbesondere auch die Steigung der Geraden mit ausreichender Genauigkeit bestimmbar ist.

Sofern auf Grund von vorher gehenden Behandlungen bereits Parameter bestimmt sind, kann aus den gemessenen Pulswellenlaufzeiten mit diesen Parametern ein Wert des Blutdrucks ermittelt werden. Die Bedingung zur Erfassung weiterer Messwertepaare kann in diesem Falle hinsichtlich des Schwellwertes auf den Blutdruck selbst als aus der gemessenen Pulswellenlaufzeit abgeleitete Größe bezogen werden.

Sofern noch keine Parameter bestimmt worden sind, kann ebenfalls ein Schwellwert vorgesehen werden, der sich auf die Pulswellenlaufzeit bestimmt. Dies kann eine absolute Differenz der Pulswellenlaufzeiten sein oder auch eine relative Abweichung der Pulswellenlaufzeiten.

Bei der vorgeschlagenen Lösung wird die Pulswellenlaufzeit bestimmt und auf den Schwellwert überwacht. Sofern die entsprechende Bedingung vorliegt, wird eine Messung des Blutdrucks mittels der Messeinheit veranlasst werden. Diese Ausgestaltung hat den Vorteil, dass es nicht notwendig ist, den Blutdruck regelmäßig zu erfassen, um beispielsweise nach Abschluss einer Messphase die Parameter bestimmen zu können. Wenn bei der vorgeschlagenen Ausgestaltung die Bedingung für die Pulswellenlaufzeit vorliegt, wird die entsprechende Messung des Blutdrucks initiiert.

Außer den so abgeleiteten Messwertepaaren können während einer Dialysebehandlung noch weitere Messwertepaare aufgenommen werden. Die Parameter der Beziehung können in diesem Fall aus einem überbestimmten Gleichungssystem ermittelt werden mittels statistischer Methoden wie beispielsweise der Methode der kleinsten Fehlerquadrate.

Bei einem Dialysegerät wird eine erste Messung vorteilhaft zu Beginn der Behandlung vorgenommen. Zu diesem Zeitpunkt ist der Blutdruck hoch, weil das Blut zur Behandlung noch nicht entnommen wurde. Der Eintritt der Bedingung der Schwellwerte der Pulswellenlaufzeit bzw. des Blutdrucks stellt sich dann während der Behandlung erwartungsgemäß von selbst ein, wenn Blut entnommen wird und dadurch der Blutdruck abfällt. Hier erweist es sich als besonders vorteilhaft, die Abhängigkeit der Bestimmung der Parameter von den Schwellwerten vorzusehen.

Es hat sich gezeigt, dass diese durch die Schwellwerte bestimmten Bedingungen mit einer ersten Messung vor der Blutentnahme und wenigstens einer weiteren Messung nach Füllung des extrakorporalen Kreislaufs mit entnommenem Blut erreicht werden.

Da ein Dialysegerät auch Mittel zum Entziehen von Flüssigkeit aus dem extrakorporalen Blut des Patienten aufweist, stellen sich die Schwellwerte erfüllende Bedingungen auch während der Dialyse ein. Gleiches gilt nach Rückgabe des extrakorporalen Blutes nach Beendigung der Dialysebehandlung. Es müssen also durchweg keine weiteren besonderen Maßnahmen getroffen werden.

Gegebenenfalls kann dazu auch auf einer Ausgabeeinheit wie einem Display oder einer Sprachausgabe die Anweisung ausgegeben werden, dass der Patient umzulagern ist (von der Rückenlage in die Seitenlage oder umgekehrt), um so eine Änderung des Blutdrucks zu bewirken. Diese Maßnahme kann zusätzlich unterstützend vorgesehen werden, wobei sich gezeigt hat, dass die Schwellwerte bereits durch die Dialysebehandlung an sich überschritten werden.

Bei der Ausgestaltung nach Anspruch 8 werden bei der Parameterbestimmung die Messwertepaare entsprechend den Zeitpunkten gewichtet berücksichtigt, zu denen die Messwertepaare aufgenommen wurden, wobei bei länger zurückliegenden Zeitpunkten, zu denen die Messwertepaare aufgenommen wurden, die Gewichtung schwächer ist.

Indem sich die Parameter mit der Zeit ändern können, wird durch die Vorgehensweise nach Anspruch 8 sichergestellt, dass aktuelle Messwerte eine stärkere Gewichtung erfahren bei der Bestimmung der Parameter. Die Parameter sind dadurch hinreichend aktuell.

Bei der Ausgestaltung nach Anspruch 9 werden zur Bestimmung der Parameter der Beziehung zwischen der Pulswellenlaufzeit und dem Blutdruck der gemessene maximale Wert des Blutdrucks sowie der gemessene minimale Wert des Blutdrucks mit den zugehörigen gemessenen Pulswellenlaufzeiten heran gezogen.

Dadurch ergibt sich eine vergleichsweise einfache Bestimmung der Parameter aus den Minimal- und Maximalwerten.

Bei der Ausgestaltung nach Anspruch 10 werden zur Bestimmung der Parameter der Beziehung nur solche Messwertepaare verwendet, die innerhalb eines bestimmten Zeitfensters ermittelt wurden.

Dies ist eine besondere Form der Gewichtung der Messwertepaare abhängig von den Zeitpunkten, zu denen die Messwertepaare ermittelt wurden. Ab einer bestimmten Zeitspanne, zu der die Messwerte zurück liegen, wird deren "Gewichtung" auf Null gesetzt, so dass diese Messwertepaare keine Berücksichtigung mehr finden.

Vorteilhaft werden mit einem Speichermedium die Daten der einzelnen Messungen für nachfolgende Behandlungen zur Verfügung gestellt.

Das Speichermedium kann magnetisch, optisch oder elektrisch sein. Vorteilhaft erweist sich hierbei, dass es sich um gängige Technologien handelt, so dass diese bei der Anwendung unmittelbar umgesetzt werden können. Das Speichermedium kann

beispielsweise ein Speichermedium sein, auf dem auch andere medizinisch relevante Daten beispielsweise über anderweitige Krankheitsbilder oder zur Organisation und finanziellen Abwicklung von Heilbehandlungskosten gespeichert werden.

Ein Ausführungsbeispiel der Erfindung wird nachfolgend näher erläutert.

Die Parameter $m$ und $n$ in Gl. (1) sind patienten- und zeitabhängig und lassen sich mit Hilfe von zwei Vergleichmessungen wie folgt ermitteln:

$$BP_{\max} = m - n \cdot PTT_{\max} \qquad (4)$$

$$BP_{\min} = m - n \cdot PTT_{\min} \qquad (5)$$

wobei

$BP_{\max}$ - gemessener maximaler Blutdruck,
$PTT_{\max}$ - $BP_{\max}$ entsprechende gemessene PTT,
$BP_{\min}$ - gemessener minimaler Blutdruck,
$PTT_{\min}$ - $BP_{\min}$ entsprechende gemessene PTT,

Aus Gl. (4) und (5) ergibt sich

$$n = \frac{BP_{\max} - BP_{\min}}{PTT_{\min} - PTT_{\max}} \qquad (6)$$

$$m = \frac{BP_{\max} \cdot PTT_{\min} - BP_{\min} \cdot PTT_{\max}}{PTT_{\min} - PTT_{\max}} \qquad (7)$$

Unter Berücksichtigung eines breiten Gültigkeitsbereichs für Gl. (1) soll die Bedingung

$$\left| BP(t_{k2}) - BP(t_{k1}) \right| \geq TH_{Bp} \qquad (8)$$

für eine Kalibrierung erfüllt werden. Hierbei ist $TH_{Bp}$ ein vorgegebener Schwellwert für den Blutdruck-Differenz, z.B. $TH_{BP}$ = 30 mmHg.

Nicht im Sinne einer abschließenden Beschränkung sondern lediglich als ein konkretes Ausführungsbeispiel ist die Beziehung zwischen der Pulswellenlaufzeit und dem Blutdruck als lineare Beziehung dargestellt.

Die Daten zur Kalibrierung können während des üblichen Ablaufs einer Hämodialysebehandlung ermittelt werden. Dabei kann eine Änderung des Blutdrucks eines Patienten durch gezielte Veränderung der Körperlage des Patienten oder durch die Modifizierung der Ultrafiltrationsrate erzeugt werden. Es ist aber auch möglich, die Blutdruckänderung des Patienten im normalen Verlauf der Dialysebehandlung zu realisieren.

Für die weitere Erläuterung wird voraus gesetzt, dass

a) ein konventioneller oszillometrischer Blutdruckmonitor mit Manschette im Dialysegerät integriert ist und
b) eine Patientenkarte zur Datenübertragung von Behandlung zu Behandlung vorhanden ist.

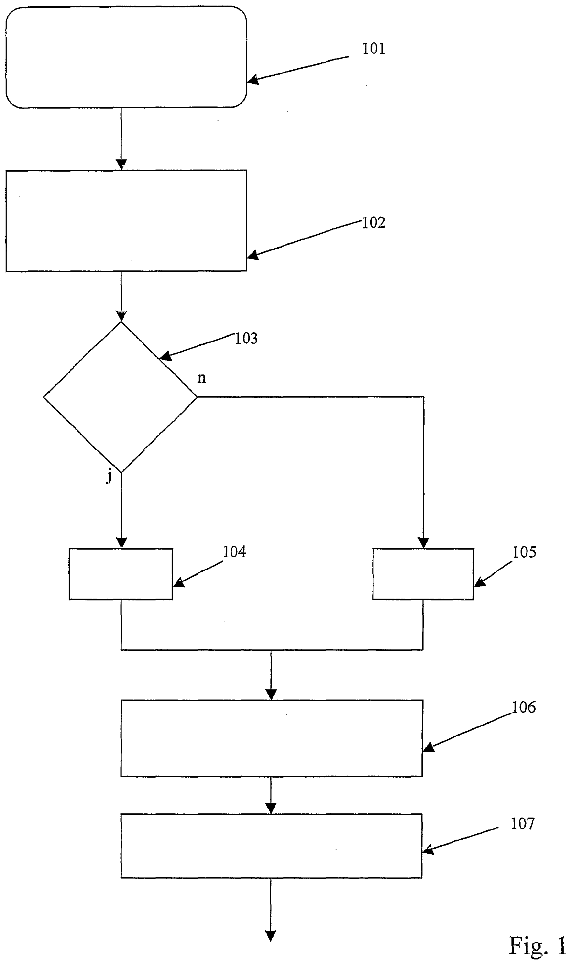

Figur 1 stellt einen Ablaufplan für die Kalibrierung der Parameter der Beziehung dar, wobei diese Beziehung in dem dargestellten Ausführungsbeispiel linear ist.

Nach einem Dialysestart entsprechend Schritt 101 werden zuerst in dem Schritt 102 die Daten von einer Patientenkarte ausgelesen und im Dialysegerät gespeichert. Die Daten beinhalten.

- $(Date_0, m_0, n_0)$ - Konstante in Gl. (1) mit Datum
- $(Date_{min0}, BP_{min0}, PTT_{min0})$ - bisher registrierter minimaler BP mit zugehöriger PTT mit Datum
- $(Date_{max0}, BP_{max0}, PTT_{max0})$ - bisher registrierter maximaler BP mit zugehöriger PTT mit Datum

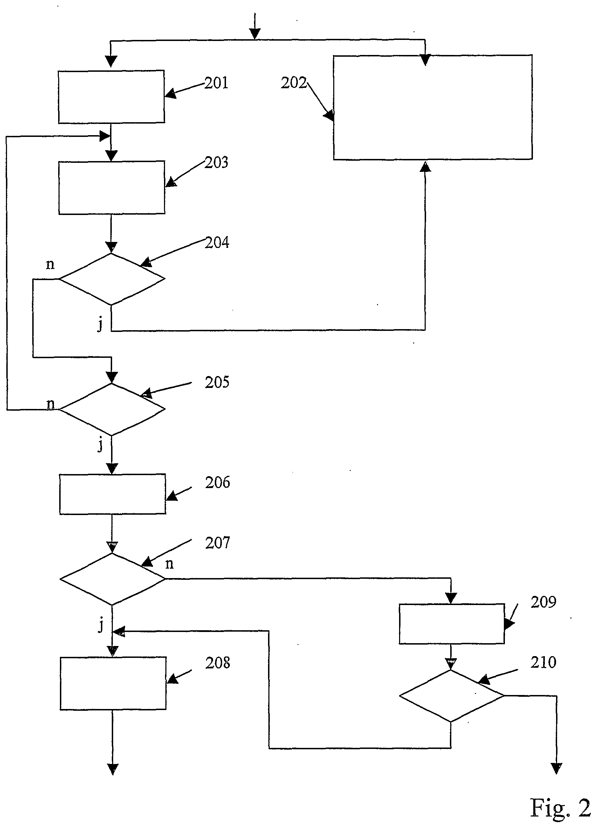

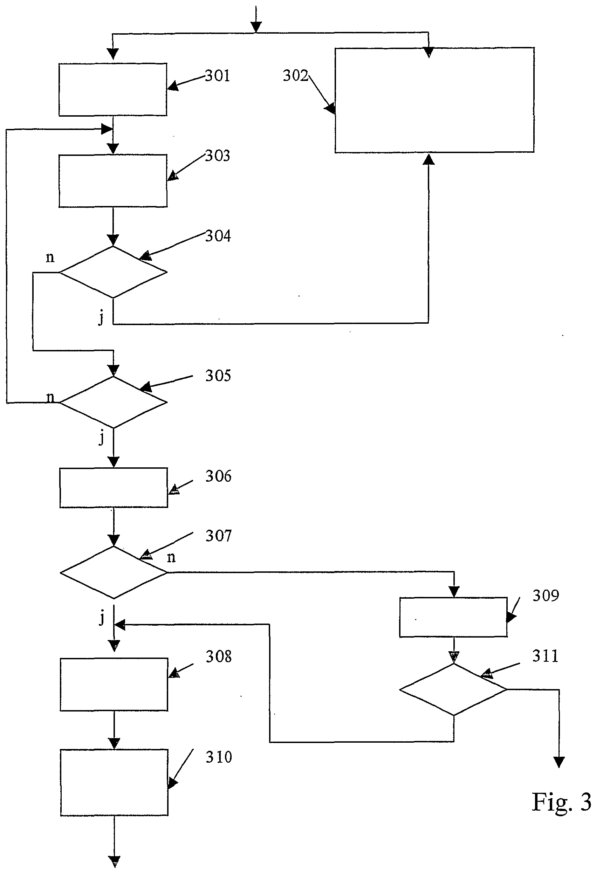

Nach dem Dialysestart erfolgt in dem Schritt 103 eine Fallunterscheidung abhängig davon, ob auf Grund vorher gehender Behandlungen die Parameter $m$ und $n$ bereits bestimmt wurden. Ist dies der Fall, wird entsprechend dem Schritt 104 mit dem weiteren Ablaufplan "B" fort gefahren, der in Figur 3 erläutert ist. Sind die Parameter m und n noch nicht bestimmt, haben diese noch den Wert 0. In diesem Fall wird bei der Überprüfung in dem Schritt 103 entsprechend dem Schritt 105 mit dem Ablaufplan "A" fort gefahren, der in Figur 2 erläutert ist.

Nachdem der entsprechende Ablaufplan "A" bzw. "B" durchlaufen wurde, erfolgt in dem Schritt 106 eine Übergabe der Parameter sowie weiterer Daten. Diese sind:

$Date_0 = Date, m_0 = m, n_0 = n$

$Date_{min0} = Date_{min}, BP_{min0} = BP_{min}, PTT_{min0} = PTT_{min}$

$Date_{max0} = Date_{max}, BP_{max0} = BP_{max}, PTT_{max0} = PTT_{max}$

mit $Date = Date_{max}$ oder $Date = Date_{min}$, abhängig davon welcher Zeitpunkt weniger weit zurück liegt.

In dem Schritt 107 werden die Kalibrierungsdaten und -ergebnisse für die Bestimmung der Parameter m und n wie folgt auf der Patientenkarte gespeichert:

$(Date_0, m_0, n_0)$

$(Date_{min0}, BP_{min0}, PTT_{min0})$

$(Date_{max0}, BP_{max0}, PTT_{max0})$.

Entsprechend dem Schritt 108 ist der Ablauf nach der Figur 1 damit beendet.

Figur 2 entspricht dem Ablaufplan, der in Figur 1 bei Schritt 105 einzusetzen ist und dort mit "A" bezeichnet wurde.

In dem Schritt 201 erfolgt eine Ermittlung eines PTT- Referenzwert ($PTT_{ref}$) in der Anfangsphase der Dialyse.

Entsprechend dem Schritt 202 erfolgt eine Datenspeicherung nach jeder oszillometrischen BP-Messung während der Dialyse

Messung 1: $(t_1, BP_1, PTT_1)$

Messung 2: $(t_2, BP_2, PTT_2)$

---

Messung n: $(t_n, BP_n, PTT_n)$

Diese Messwertepaare werden zum einen (parallel zu dem Ablauf der Überprüfung in den Schritten 201, 203, 204) in zyklischen Abständen von beispielsweise 30 Minuten aufgenommen. Außerdem kann sich auf Grund der Überprüfung in dem Schritt 204 ergeben, dass ein solches Messwertepaar aufgenommen werden soll. Die Überprüfung in dem Schritt 204 initiiert dann die Messung des Blutdrucks zu diesem Zeitpunkt.

Entsprechend dem Schritt 203 erfolgt eine Bestimmung der relativen PTT-Änderung nach Gl. (9) für jede neue PTT-Wert PTT(i)

$$relPttChg = \frac{\left| PTT_{Ref} - PTT(i) \right|}{PTT_{Ref}} \cdot 100 \qquad (9)$$

In dem Schritt 204 wird überprüft, ob eine Schwellwertüberschreitung durch die PTT-Änderung vorliegt. In dem vorliegenden Ausführungsbeispiel erfolgt dieses Überprüfung durch eine Bestimmung der relativen Änderung, es kann aber auch eine Überprüfung durch eine Bestimmung der absoluten Änderung vorgenommen werden:

$$relPttChg \geq TH_{PttChg} \qquad (10)$$

wobei $TH_{PttChg}$ - vorgegebener Schwellwert für die relative PTT-Änderung.

Eine Schellwertüberschreitung signalisiert den Eintritt eines BP-Abfalls, wobei entsprechend dem Schritt 202 eine oszillometrische BP-Messung aktiviert und das Ergebnis gespeichert wird.

Wird bei der Überprüfung in dem Schritt 204 nicht erkannt, dass der Schwellwert überschritten wurde, erfolgt ein Übergang zu dem Schritt 205, in dem überprüft wird, ob die Behandlung beendet wurde.

Ist dies nicht der Fall, erfolgt ein Übergang zu dem Schritt 203, in dem wiederum die Änderung des PTT überprüft wird.

Wurde ein Ende der Behandlung erkannt, erfolgt ein Übergang zu dem Schritt 206. Es werden maximale und minimale BP-Werte und die entsprechende PTT-Werte aus den gespeicherten Daten ermittelt:

$$BP_{\min 1} = \mathrm{Min}(BP_1, BP_1, \cdots, BP_n) \qquad => \qquad PTT_{\min 1} = PTT\big|_{BP=BP\min 1}$$

$$BP_{\max 1} = \mathrm{Max}(BP_1, BP_1, \cdots, BP_n) \qquad => \qquad PTT_{\max 1} = PTT\big|_{BP=BP\max 1}$$

Im vorliegenden Ausführungsbeispiel erfolgt eine Bestimmung der Parameter mittels zweier Messwertepaare: $(Date_{\max 1}, BP_{\max 1}, PTT_{\max 1})$ und $(Date_{\min 1}, BP_{\min 1}, PTT_{\min 1})$

Nach der Variablenübergabe

$Date_{\min} = Date_{\min 1}$, $BP_{\min} = BP_{\min 1}$, $PTT_{\min} = PTT_{\min 1}$
$Date_{\max} = Date_{\max 1}$, $BP_{\max} = BP_{\max 1}$, $PTT_{\max} = PTT_{\max 1}$

wird in dem Schritt 207 geprüft, ob die Bedingungen

$$BP_{\max} - BP_{\min} \geq TH_{Bp}$$

$$PTT_{\min} - PTT_{\max} \geq TH_{Ptt}$$

erfüllt sind. Hierbei ist $TH_{Ptt}$ ein vorgegebener Schwellwert für die PTT-Differenz als absolute Abweichung der Pulswellenlaufzeit und $TH_{Bp}$ ein vorgegebener Schwellwert für die Blutdruckdifferenz als absolute Abweichung des Blutdrucks. Statt absoluter Abweichungen können aber auch relative Abweichungen zu Anwendung kommen.

Wird in dem Schritt 207 fest gestellt, dass diese Bedingungen erfüllt sind, wird entsprechend dem Schritt 208 eine Bestimmung der Parameter durchgeführt, indem die Parameter $m$ und $n$ in Gl. (1) nach Gl. (7) und (6) berechnet werden.

Wird bei der Überprüfung in dem Schritt 207 fest gestellt, dass mindestens eine der beiden Bedingungen nicht erfüllt ist, wird im Rahmen des vorliegenden Ausführungsbeispiel darauf geschlossen, dass die Daten aus der aktuellen Behandlung für eine Parameterbestimmung nicht ausreichend geeignet sind. Es folgt dann entsprechend dem Schritt 209 eine "Datenoptimierung" durch

$BP_{\min} = \mathrm{Min}(BP_{\min 0}, BP_{\min 1})$, $PTT_{\min}$, $Date_{\min}$
$BP_{\max} = \mathrm{Max}(BP_{\max 0}, BP_{\max 1})$, $PTT_{\max}$, $Date_{\max}$

Dabei erfolgt ein Datenabgleich mit Daten voran gegangener Messungen, die beispielsweise auf einer Patientenkarte gespeichert sein können. Dabei wird zumindest der Werte-Tripel der aktuellen Messung entnommen, um die Aktualität der Messwerte sicher zu stellen.

Danach werden entsprechend dem Schritt 210 mit den neu bestimmten Daten die Bedingungen entsprechend dem Schritt 207 nochmals geprüft. Zusätzlich wird ein dritter Schwellwert $TH_{Date}$ wegen der Zeitvariabilität von Gl. (1) eingefügt:

$$\big|Date_{\max} - Date_{\min}\big| \leq TH_{Date}$$

Ergibt die Überprüfung in dem Schritt 210, dass die Bedingungen mit den neuen Daten erfüllt werden, wird eine Bestimmung der Konstanten $m$ und $n$ in Gl. (1) nach Gl. (7) und (6) vorgenommen.

Ergibt die Überprüfung in dem Schritt 210, dass die Bedingungen auch mit den neuen Daten noch nicht erfüllt sind, wird der Ablauf beendet, ohne dass eine Bestimmung der Parameter vorgenommen wird.

Die Figur 3 betrifft eine Darstellung, die in Figur 1 an dem Schritt 104 einzusetzen ist und dort mit "B" bezeichnet wurde.

Die Schritt 301 - 308 in dem Ablaufplan der Figur 3 entsprechen den entsprechenden Schritten 201 - 208 in dem Ablaufplan der Figur 2. Der einzige Unterscheid besteht in dem vorliegenden Ausführungsbeispiel darin, dass bereits Werte für die Parameter m und n vorliegen, so dass für die Überprüfung des Schwellwertes in der Figur 304 aus den PTT-Werten mit den bereits bestimmten Parametern m und n unmittelbar Blutdruckwerte ermittelt werden können. Die Schwellwerte können also unmittelbar auf den Blutdruck bezogen werden.

Es ist zu sehen, dass dem Schritt 308 noch ein weiterer Schritt 310 nachfolgt. In diesem Schritt kann eine Neubestimmung der Parameter m und n vorgenommen werden. In diesem Schritt erfolgt eine Neubestimmung der Parameter m und n, indem die aus der aktuellen Behandlung bestimmten Parameter m und n gewichtet mit den bisher bestimmten Parametern m0 und n0 zusammen gefasst werden entsprechend den nachfolgenden Gleichungen 14 und 15:

$$m = \alpha \cdot m + (1 - \alpha) \cdot m_0 \qquad 0 \leq \alpha \leq 1 \qquad (14)$$

$$n = \beta \cdot n + (1 - \beta) \cdot n_0 \qquad 0 \leq \beta \leq 1 \qquad (15)$$

wobei $\alpha$ und $\beta$ zwei vorgegebene Parameter sind, die selbst abhängig von anderen Größen sein können, z. B. von der Zeit oder auch von den jeweiligen Druckdifferenzen.

Wird bei der Überprüfung in dem Schritt 307 fest gestellt, dass wenigstens eine der Bedingungen nicht erfüllt ist, wird im Rahmen des vorliegenden Ausführungsbeispiels darauf geschlossen, dass die Daten aus der aktuellen Behandlung für sich allein für die Parameterbestimmung nicht ausreichend sind.

Es folgt dann in dem Schritt 309 eine "Datenoptimierung" durch

$$BP_{\min} = \text{Min}(BP_{\min0}, BP_{\min1}), PTT_{\min}, \text{Date}_{\min}$$
$$BP_{\max} = \text{Max}(BP_{\max0}, BP_{\max1}), PTT_{\max}, \text{Date}_{\max}$$

Dabei erfolgt ein Rückgriff auf Messwertepaare, die bei vorher gehenden Behandlungen gewonnen wurden. Berücksichtigt werden dabei lediglich Messwertepaare, die zu einem Zeitpunkt aufgenommen wurden, der innerhalb eines bestimmten Zeitfensters liegt. Bei den Messwertepaaren wird dann der Minimalwert des Blutdrucks sowie der Maximalwert des Blutdrucks ermittelt. Mit diesen Messwerten wird dann nochmals entsprechend dem Schritt 307 überprüft, ob die Bedingungen vorliegen. Ist dies dann der Fall, erfolgt ein Übergang zu dem Schritt 308.

**Patentansprüche**

1. Dialysegerät mit
   einer Messeinheit zur Erfassung des Blutdrucks;
   einer Messeinrichtung zur Erfassung einer Pulswellenlaufzeit;
   sowie einer Auswerteeinheit;

   wobei das Dialysegerät dazu ausgelegt ist, Messwertpaare zu bilden, wobei ein Messwert der Messeinheit und ein zeitgleicher Messwert der Messeinrichtung ein Messwertpaar darstellen; und
   wobei das Dialysegerät dazu ausgelegt ist, zwei dieser Messwertpaare zu Zeitpunkten zu ermitteln, bei denen die absolute und/oder die relative Abweichung der Pulswellenlaufzeit und/oder des Blutdrucks oberhalb eines Schwellwertes liegt; und
   wobei das Dialysegerät dazu ausgelegt ist, ein erstes der zwei Messwertpaare vor einer Blutentnahme zu Beginn einer Dialysebehandlung zu ermitteln und ein zweites der zwei Messwertpaare nach Füllung eines extrakorporalen Kreislaufs mit entnommenem Blut zu ermitteln; und
   wobei die Auswerteeinheit dazu konfiguriert ist, um Parameter (m, n) einer Beziehung zwischen der Pulswellenlaufzeit und dem Blutdruck auf Basis der beiden Messwertpaare zu bestimmen.

2. Dialysegerät nach Anspruch 1, **dadurch gekennzeichnet, dass** bei der Parameterbestimmung die Messwertepaare entsprechend den Zeitpunkten gewichtet berücksichtigbar sind (310), zu denen die Messwertepaare aufgenommen wurden, wobei bei länger zurückliegenden Zeitpunkten, zu denen die Messwertepaare aufgenommen wurden, die Gewichtung schwächer ist.

**3.** Dialysegerät nach Anspruch 1 oder 2, **dadurch gekennzeichnet, dass** zur Bestimmung der Parameter der Beziehung zwischen der Pulswellenlaufzeit und dem Blutdruck der gemessene maximale Wert des Blutdrucks sowie der gemessene minimale Wert des Blutdrucks mit den zugehörigen gemessenen Pulswellenlaufzeiten heranziehbar sind (208, 308).

**4.** Dialysegerät nach einem der Ansprüche 1 bis 3, **dadurch gekennzeichnet, dass** zur Bestimmung der Parameter der Beziehung nur solche Messwertepaare verwendbar sind, die innerhalb eines bestimmten Zeitfensters ermittelt wurden (309).

**5.** Dialysegerät nach einem der vorher gehenden Ansprüche, **dadurch gekennzeichnet, dass** dem Dialysegerät eine Dateneinleseeinheit zugeordnet ist bzw. zuordnbar ist, mit der der Auswerteeinheit Messwertepaare der Messeinheit und der Messeinrichtung von früheren Behandlungen desselben Patienten von einem Speichermedium zugeführt werden.

**6.** Dialysegerät nach Anspruch 5, **dadurch gekennzeichnet, dass** die Dateneinleseeinheit zugleich auch eine Datenausleseeinheit ist, mit der Messwertepaare der aktuellen Behandlung auf das Speichermedium geschrieben werden.

**7.** Verfahren zur Bestimmung der Parameter der Beziehung zwischen einer Pulswellenlaufzeit und dem Blutdruck, wobei mittels einer Messeinheit der Blutdruck und mittels einer Messeinrichtung die Pulswellenlaufzeit ermittelt werden,

> wobei Messwertpaare gebildet werden, wobei ein Messwert der Messeinheit und ein zeitgleicher Messwert der Messeinrichtung ein Messwertpaar darstellen; und
> wobei zwei dieser Messwertpaare zu Zeitpunkten ermittelt werden, bei denen die absolute und/oder die relative Abweichung der Pulswellenlaufzeit und/oder des Blutdrucks oberhalb eines Schwellwertes liegt; und
> wobei ein erstes der zwei Messwertpaare vor einer Blutentnahme zu Beginn einer Dialysebehandlung und ein zweites der zwei Messwertpaare nach Füllung eines extrakorporalen Kreislaufs mit entnommenem Blut ermittelt wird; und
> wobei Parameter (m, n) der Beziehung zwischen der Pulswellenlaufzeit und dem Blutdruck auf Basis der beiden Messwertpaare bestimmt werden.

**8.** Verfahren nach Anspruch 7, **dadurch gekennzeichnet, dass** bei der Parameterbestimmung die Messwertepaare entsprechend den Zeitpunkten gewichtet berücksichtigt werden (310), zu denen die Messwerte paare aufgenommen wurden, wobei bei länger zurückliegenden Zeitpunkten, zu denen die Messwertepaare aufgenommen wurden, die Gewichtung schwächer ist.

**9.** Verfahren nach Anspruch 7 oder 8, **dadurch gekennzeichnet, dass** zur Bestimmung der Parameter der Beziehung zwischen der Pulswellenlaufzeit und dem Blutdruck der gemessene maximale Wert des Blutdrucks sowie der gemessene minimale Wert des Blutdrucks mit den zugehörigen gemessenen Pulswellenlaufzeiten heran gezogen werden (208, 308).

**10.** Verfahren nach einem der Ansprüche 7 bis 9, **dadurch gekennzeichnet, dass** zur Bestimmung der Parameter der Beziehung nur solche Messwertepaare verwendet werden, die innerhalb eines bestimmten Zeitfensters ermittelt wurden (309).

**Claims**

**1.** Dialyzer, comprising
a blood-pressure measuring unit;
a pulse-wave-transit-time measuring system; as well as
an evaluation unit,
wherein the dialyzer is configured to create pairs of measured values,
wherein a measured value of the measuring unit and a simultaneous measured value of the measuring system form a pair of measured values; and
wherein the dialyzer is configured to determine two of these pairs of measured values at time points at times when the absolute and/or the relative deviation of the pulse wave transit time and/or of the blood pressure is above a threshold value; and

wherein the dialyzer is configured to determine a first of the two pairs of measured values prior to a withdrawal of blood at the start of a dialysis treatment, and to determine a second of the two pairs of measured values after the filling of an extracorporeal circuit with withdrawn blood; and

wherein the evaluation unit is configured to determine parameters describing the relationship between the pulse wave transit time and the blood pressure on the basis of the two pairs of measured values.

2. Dialyzer according to claim 1, **characterized in that** for parameter determination, the pairs of measured values can be weighted according to the times at which the pairs of measured values were recorded, the weighting being weaker for pairs of measured values recorded further back in time.

3. Dialyzer according to claim 1 or 2, **characterized in that** the maximum and minimum blood pressures measured, together with the corresponding measured pulse wave transit times, can be used to determine the parameters describing the relationship between the pulse wave transit time and the blood pressure (208, 308).

4. Dialyzer according to one of the claims 1 to 3, **characterized in that** only such pairs of measured values as are obtained within a given time window can be used to determine the parameters describing the relationship (309).

5. Dialyzer according to one of the preceding claims, **characterized in that** a data input unit is assigned or assignable to the dialyzer, said input unit serving to supply pairs of measured values, obtained by the measuring unit and the measuring system during earlier therapy sessions with the same patient, to the evaluation unit from a storage medium.

6. Dialyzer according to claim 5, **characterized in that** the data input unit simultaneously is a data output unit with which the pairs of measured values from the current therapy session are written onto the storage medium.

7. Method of determining the parameters describing the relationship between a pulse-wave-transit-time and the blood pressure, wherein the blood pressure can be determined via a measuring unit, and the pulse-wave-transit-time can be determined via a measuring system,

wherein pairs of measured values are formed, wherein a measured value of the measuring unit and a simultaneous measured value of the measuring system constitute a pair of measured values; and

wherein two of these pairs of measured values are determined at times when the absolute and/or relative deviation of the pulse-wave-transit-time and/or of the blood pressure are above a threshold value; and

wherein a first one of the two pairs of measured values is determined prior to a withdrawal of blood at the start of a dialysis treatment, and a second one of the two pairs of measured values is determined after the filling of an extracorporeal circuit with withdrawn blood; and

wherein parameters (m, n) describing the relationship between the pulse wave transit time and the blood pressure are determined on the basis of the two pairs of measured values.

8. Method according to claim 7, **characterized in that** for parameter determination, the pairs of measured values are weighted (310) according to the times at which the pairs of measured values were recorded, the weighting being weaker for pairs of measured values recorded further back in time.

9. Method according to claim 7 or 8, **characterized in that** the maximum and minimum blood pressures measured, together with the corresponding measured pulse wave transit times, are used to determine the parameters describing the relationship between the pulse wave transit time and the blood pressure (208, 308).

10. Method according to one of the claims 7 to 9, **characterized in that** only such pairs of measured values as are obtained within a given time window are used to determine the parameters describing the relationship (309).

**Revendications**

1. Appareil de dialyse, comprenant :

une unité de mesure pour la détection de la pression artérielle ;
un dispositif de mesure pour la détection d'une temps de propagation d'une onde de pouls ;
ainsi qu'une unité d'évaluation ;
dans lequel l'appareil de dialyse est configuré à former des paires de valeur de mesure, dans lequel une valeur de mesure de l'unité de mesure et une valeur de mesure simultanée du dispositif de mesure représentent une paire de valeur de mesure ; et

dans lequel l'appareil de dialyse est configuré pour détecter deux de ces paires de valeur de mesure à des moments donnés, auxquels l'écart absolu et/ou relative du temps de propagation d'une onde de pouls et/ou de la pression artérielle est supérieure à une valeur de seuil ; et

dans lequel l'appareil de dialyse est configuré pour identifier une première paire des deux paires de valeur de mesure avant une prise de sang au début d'un traitement de dialyse et pour identifier une deuxième des deux paires de valeur de mesure après avoir remplis un circuit extracorporel avec du sang prélevé ; et

dans lequel l'unité d'analyse est configurée pour déterminer des paramètres (m, n) d'un rapport entre le temps de propagation d'une onde de pouls et la pression artérielle sur la base des deux paires de valeur de mesure.

2. Appareil de dialyse selon la revendication 1, **caractérisé en ce que** lors de la détermination du paramètre, les paires de valeur de mesure peuvent être considérées de manière pondérée, correspondant aux moments donnés (310) auxquels les paires de valeur de mesure ont été enregistrées, dans lequel, à des moments donnés reposant longtemps dans le passé, auxquels les paires de valeur de mesure ont été enregistrées, la pondération est plus faible.

3. Appareil de dialyse selon la revendication 1 ou 2, **caractérisé en ce que** pour la détermination des paramètres du rapport entre le temps de propagation d'une onde de pouls et la pression artérielle, la valeur maximale mesurée de la pression artérielle ainsi que la valeur minimale mesurée de la pression artérielle peuvent être utilisées (208,309) avec les temps de propagation d'une onde de pouls mesurés et associés.

4. Appareil de dialyse selon l'une quelconque des revendications 1 à 3, **caractérisé en ce que** pour identifier des paramètres pour le rapport, seulement de telles paires de valeur de mesure peuvent être utilisées, qui ont été détectées dans une fenêtre temporelle définie (309).

5. Appareil de dialyse selon l'une quelconque des revendications précédentes, **caractérisé en ce qu'**une unité d'enregistrement des données est attribuée ou peut être attribuée à l'appareil de dialyse, avec laquelle des paires de valeur de mesure de l'unité de mesure et du dispositif de mesure d'anciens traitements d'un même patient sont fournies à l'unité d'évaluation, à partir d'un support de stockage.

6. Appareil de dialyse selon la revendication 5, **caractérisé en ce que** l'unité de d'entrée de données est également une unité de sortie de données, avec laquelle les paires de valeur de mesure du traitement actuelle sont écrites sur le support de stockage.

7. Procédé pour la détermination des paramètres pour le rapport entre un temps de propagation d'une onde de pouls et la pression artérielle, dans lequel la pression artérielle est identifiée au moyen d'une unité de mesure et le temps de propagation d'une onde de pouls est identifié au moyen d'une unité de mesure,

dans lequel les paires de valeur de mesure sont formées, dans lequel une valeur de mesure de l'unité de mesure et une valeur de mesure simultanée du dispositif de mesure, représentent une paire de valeur de mesure ; et

dans lequel deux de ces paires de valeur de mesure sont identifiées à des moments donnés, auxquels l'écart absolu et/ou relatif du temps de propagation d'une onde de pouls et/ou de la pression artérielle est supérieur à une valeur de seuil ; et

dans lequel une première paire des deux paires de valeur de mesure est identifiée avant une prise de sang au début d'un traitement de dialyse, et une deuxième des deux paires de valeur de mesure est identifiée après avoir remplis un circuit extracorporel avec du sang prélevé, et

dans lequel des paramètres (m, n) du rapport entre le temps de propagation d'une onde de pouls et la pression artérielle sont déterminés sur la base des deux paires de valeur de mesure.

8. Procédé selon la revendication 7, **caractérisé en ce que** lors de la détermination du paramètre les paires de valeur de mesure sont considérées de manière pondérée des moments donnés respectives (310), auxquels les paires de valeur de mesure ont été enregistrées, dans lequel à des moments donnés reposant longtemps dans le passé, auxquels les paires de valeur de mesure sont enregistrées, la pondération est plus faible.

9. Procédé selon la revendication 7 ou 8, **caractérisé en ce que** pour la détermination des paramètres du rapport entre le temps de propagation d'une onde de pouls et la pression artérielle, la valeur maximale mesurée de la pression artérielle ainsi que la valeur minimale mesurée de la pression artérielle sont utilisées (208, 308) avec les temps de propagation d'une onde de pouls mesurés associés.

**10.** Procédé selon l'une des revendications précédentes 7 à 9, **caractérisé en ce que** pour identifier des paramètres pour le rapport, seulement de telles paires de paramètre sont utilisées, qui ont été détectées (309) dans une fenêtre temporelle définie.

Fig. 1

Fig. 2

Fig. 3

**IN DER BESCHREIBUNG AUFGEFÜHRTE DOKUMENTE**

**In der Beschreibung aufgeführte Patentdokumente**

- US S6736789 B
- US 20020193691 A1
- US 5876248 A
- DE 10114383 A1

- US 6852083 B
- US 5603329 A
- US 5876348 A
- EP 0875200 A1